(19) Europäisches Patentamt
European Patent Office
Office européen des brevets

(11) EP 1 581 390 B2

(12) **NEW EUROPEAN PATENT SPECIFICATION**
After opposition procedure

(45) Date of publication and mention of the opposition decision:
**31.08.2016 Bulletin 2016/35**

(45) Mention of the grant of the patent:
**27.04.2011 Bulletin 2011/17**

(21) Application number: **04700829.7**

(22) Date of filing: **08.01.2004**

(51) Int Cl.:
***A61F 13/15*** (2006.01) ***B32B 27/32*** (2006.01)

(86) International application number:
**PCT/US2004/000280**

(87) International publication number:
**WO 2004/063270 (29.07.2004 Gazette 2004/31)**

(54) **ELASTIC ARTICLES AND PROCESSES FOR THEIR MANUFACTURE**
ELASTISCHE ARTIKEL UND HERSTELLUNGSVERFAHREN DAFÜR
ARTICLES ÉLASTIQUES ET LEURS PROCÉDÉS DE FABRICATION

(84) Designated Contracting States:
**AT BE BG CH CY CZ DE DK EE ES FI FR GB GR HU IE IT LI LU MC NL PT RO SE SI SK TR**

(30) Priority: **08.01.2003 US 438750 P**

(43) Date of publication of application:
**05.10.2005 Bulletin 2005/40**

(73) Proprietor: **ExxonMobil Chemical Patents Inc.**
**Baytown, TX 77520 (US)**

(72) Inventor: **SRINIVAS, Srivatsan**
**Pearland, TX 77584 (US)**

(74) Representative: **ExxonMobil Chemical Europe Inc. et al**
**IP Law Europe**
**Hermeslaan 2**
**1831 Machelen (BE)**

(56) References cited:

**EP-A- 0 533 493**
**WO-A1-00/01745**
**WO-A2-02/051928**
**WO-A2-03/040202**
**US-A- 4 714 735**
**US-A- 5 344 691**
**US-A- 5 366 796**
**US-A- 5 716 698**
**US-A- 5 932 157**
**US-A- 5 932 157**
**US-A1- 5 620 803**
**US-A1- 2002 182 426**
**US-A1- 2002 182 426**
**US-A1- 2002 182 426**
**US-B1- 6 423 420**
**US-B1- 6 635 717**



Printed by Jouve, 75001 PARIS (FR)

## Description

### FIELD OF THE INVENTION

The present invention relates to articles, such as films, fabrics, and fibers, which can become more elastic by an irreversible mechanical process, and processes for their manufacture. The present invention also relates to processes in which elasticity is imparted to articles, and articles that have undergone such processes.

### BACKGROUND

Known coextrusion processes involve melting of at least two separate polymer compositions and their simultaneous extrusion and immediate combination. The extrudate can be cooled until the polymers have solidified and can be mechanically would onto a roll, Winding the extrudate around a chilled roll may accelerate the cooling. The extrudate may be oriented to a controlled degree in the machine and/or transverse direction. This drawing may be performed at temperatures below the melting point of the coextrudate. In this way articles can be made combining the desired properties of different polymer compositions.

Coextruded films are generally made from polymer compositions, which develop considerable mechanical strength upon cooling by the forming of crystalline phases. Such polymer compositions are also capable of developing increased strength upon orientation of the compositions and better alignment of the crystalline regions.

Elasticity in films is desired for a number of applications. Examples of such applications are in personal care products, such as diaper back sheets, diaper waistbands, and diaper ears; medical applications, such as gowns and bags; and garment applications, such as disposable wear. In use in the final structure, elastic articles can provide desirable characteristics, such as helping to achieve compliance of garments to an underlying shape. In diaper waistbands, for example, a high elastic recovery ensures the return of the waistband to its original shape throughout the use of the diaper.

Elasticity is generally obtained from the use of amorphous elastomeric polymer compositions. There are, however, many difficulties and problems associated with the processing of such polymer compositions into articles such as films and fibers. For example, elasticity limits the line speed, particularly during processing at high line speeds, because the tension applied to the film causes the film to extend, sometimes in an unstable manner. Furthermore, elastic polymers are generally high molecular weight amorphous polymers that can be difficult to process into articles such as films, fabrics and fibers. A further difficulty in processing elastic films arises from the tackiness of the films on the roll, which causes "blocking", i.e., sticking of the film to itself. This limits the storage of the article after it has been produced. Elastic polymers can also have poor aesthetics, including, for example, poorsurface appearance and rubbery/tacky feel or touch.

Several approaches have been taken to alleviate these problems. U.S. Patent No. 6,649,548 and references therein disclose laminates of nonwoven fabrics with films to impart a betterfeel. U.S. Patent Nos.4,629,643 and 5,814,413 and PCT Publications WO99/47339 and WO01/05574 disclose various mechanical and processing techniques used to emboss ortexture the film surface in orderto increase the surface area and improve the feel. U.S. Patent Nos. 4,714,735 and 4,820,590 disclose films comprising an elastomer, ethylene vinyl acetate (EVA), and process oil that are prepared by orienting the film at elevated temperature and annealing the film to freeze in the stresses. The film is subsequently heated, which shrinks and forms an elastic film. In one embodiment, these references also disclose films having layers of ethylene polymers or copolymers on either side of the elastic film to reduce tackiness. By heat-setting the film it can be stabilized in its extended condition. Upon application of heat higher than the heat setting temperature, the heat set is removed and the film returns to its original length and remains elastic. Two heating steps are involved, adding cost and complexity. U.S. Patent No. 4,880,682 discloses a multilayer film comprising an elastomer core layer and thermoplastic skin layer(s). The elastomers used were ethylene propylene (EP) rubbers, ethylene propylene diene monomer rubbers (EPDM), and butyl rubber, in a laminated structure with EVA as the skin layers. After casting, these films were oriented to yield films having a microundulated surface providing a low gloss film. Microtextured elastomeric laminated films having at least one adhesive layer are disclosed in U.S. Patent Nos. 5,354,597 and 5,376,430. U.S. Patent No. 4,476,180 describes blends of styrenic block copolymer based elastomers with ethylene-vinyl acetate copolymers to reduce the tackiness without excessively degrading the mechanical properties.

In some embodiments, the present invention provides an elastic material having one or more of the following advantages over known materials: improved elasticity; better processing (for example, compared to EP, EPDM, and styrenic block copolymers); reduction in tackiness which provides the ability to store the material before further use or processing; and desirable surface appearance and feel, such that there is no need to bond nonwoven fabrics or use mechanical techniques to texture or emboss the surface.

## SUMMARY OF THE INVENTION

The present invention concerns an article comprising:

(a) a low crystallinity layer comprising a low crystallinity polymer, the upper limit of the melting point by DSC of the low crystallinity polymer being 110°C; and
(b) a high crystallinity layer plastically deformed by elongation comprising a high crystallinity polymer, wherein said high crystallinity polymer has:

a melting point as determined by DSC which is at least 25°C higher than that of said low crystallinity polymer;

wherein said low crystallinity polymer and said high crystallinity polymer have compatible stereoregular polypropylene crystallinity, and said low crystallinity layer is in contact with said plastically deformed high crystallinity layer, and the article has a Haze value of greater than 70%, and a load loss of less than 70%.

Further, the present invention concerns a garment portion, preferably a diaper backsheet, comprising the article adhered to a substrate.
The invention also concerns a process for making the article. The process comprises forming the article and elongating the article.

## BRIEF DESCRIPTION OF THE DRAWINGS

Figure 1 is a stress-strain plot of unstretched and elongated films of one embodiment of the invention.
Figure 2 is a graph of the load-loss and permanent set of unstretched and elongated films of one embodiment of the invention.
Figures 3A, 3B, and 3C are photographs showing a film according to one embodiment before (Figure 3A) and after (Figures 3B and 3C) elongation.

## DETAILED DESCRIPTION

The terms "low crystallinity" and "high crystallinity" are used herein in a relative and not an absolute sense.

### The Article

The invention includes an article comprising a low crystallinity layer and a high crystallinity layer, wherein the high crystallinity layer is plastically deformed by elongation. The term "elongation" is defined herein to be uniaxially orbiaxially elongating the article to a degree sufficient to cause plastic deformation of the high crystallinity layer. The degree of elongation sufficient to cause plastic deformation can be readily determined by one skilled in the art as described below. Whether an article, or a portion thereof, is plastically deformed can also be readily determined by one skilled in the art as described below, including surface roughness and increase in Haze values.
The initial article has poor elastic and hysteresis characteristics due to the influence of the high crystallinity layer(s). However, upon elongating the article beyond the plastic deformation point of the high crystallinity layer(s), the elastic and hysteresis properties are improved, as illustrated in Figure 1. The hysteresis load loss, defined here as the percentage of load loss during retraction compared to the stretching cycle, prior to elongation is less than 70%, and a corresponding tension set is less than 20% after elongation to 100%.
Dimensional profile (surface roughness) and increase in Haze values can be used by one of ordinary skill in the art to determine whether an article is plastically deformed. Haze was measured according to ASTM D1003 using a HazeGard PLUS Hazemeter available from BYK Gardner of Melville, New York, with a light source CIE illuminant C. Plastically deformed articles according to the invention have a Haze value of greater than 70%, or greater than 80%, or greater than 90%. The plastically deformed articles have an increased haze value compared to the article prior to elongation. The change (increase) in haze, ΔHaze, can be characterized by:

$$\Delta Haze\ (\%) = Haze_{final}\ (\%) - Haze_{initial}\ (\%)$$

wherein $Haze_{final}$ and $Haze_{initial}$ are the haze values after and before elongation, respectively. Articles according to the invention can have ΔHaze values of greater than 0%, or greater than 10%, or greater than 25%, or greater than 50% or greater than 70%. In a particular embodiment, the $Haze_{initial}$ of Film 1 in the examples below was 7% and the $Haze_{final}$ of Flm 1 after plastic deformation was 91%, giving a ΔHaze value of 84%.

The surface roughness of the article can be measured by a number of instruments capable of precise surface roughness measurements. One such instrument is Surfcom® 110B manufactured by Tokyo Seimitsu Company. The Surfcom® instrument contained a diamond stylus which moves across the surface of the sample. This sample can range in hardness from metal to plastic to rubber compounds. The instrument records the surface irregularities over the length travelled by the stylus. The surface roughness is quantified using a combination of three factors -

Ra (μm) - the arithmetic mean representing the departure of the extrudate surface profile from a mean line;
Ry (μm) - the sum of the height of the highest peak from a mean line and the depth of the deepest valley from a mean line; and
$R_2$ (μm) - the Sum of two means, which are the average height of the five highest peaks from a mean line and the average depth of the five deepest valleys from a mean line.

The combination of the Ra, Ry and Rz values characterize the surface profile of the film. By comparing these values for the unelongated films versus the plastically deformed films in the examples below, it is concluded that the roughness increased as a result of the orientation process.

In some embodiments, the article is elongated in at least one direction to an elongation of at least 150% or at least 200% of its original length or width. Generally, the article is elongated at a temperature below the melting temperature of either of the low crystallinity polymer or high crystallinity polymer.

In a particular embodiment, the article is formed by coextruding the low crystallinity layer and high crystallinity layer prior to elongation. The article can optionally be oriented in the machine direction (MD) or the transverse direction (TD) or both directions (biaxially) using conventional equipment and processes. Orientation can be carried in a separate step priorto the elongation step described below. Thus, an oriented article can be prepared as an intermediate product, which is then later elongated in a separate step. In this embodiment, the orientation is preferably carried out such that minimal plastic deformation of the high crystallinity layer occurs. Alternatively, orientation and elongation to plastic deformation can be carried out in a single step.

In the article according to the present invention, the low crystallinity layer is in contact with the high crystallinity layer. The term "in contact" is defined herein to mean that there is sufficient interfacial adhesion provided by, for example, compatible crystallinity, such that there is no delamination between adjacent layers of polymers, even after orientation and/or elongation. In some embodiments, the low crystallinity layer is adhered to the high crystallinity layer using conventional materials, such as adhesives.

In one embodiment, the article is a film wherein the high crystallinity layer forms a skin layer. In another embodiment, the high crystallinity layer is intermediate to the low crystallinity layer and another type of skin layer, such as any conventional polymer layer. In yet another embodiment, high crystallinity, layers are present on both sides of the low crystallinity layer. In this embodiment, the two high crystallinity layers can be the same or different in composition and the same or different in thickness. In yet another embodiment, the article includes, in sequence, a high crystallinity layer, a low crystallinity layer, and an additional low crystallinity layer. In this embodiment, the two low crystallinity layers can be the same or different in composition and the same or different in thickness. It should be appreciated that the article can comprise as many layers as desired.

The high crystallinity layer or one or multiple low crystallinity layers may also form a skin layer and be adapted to adhere by melting onto a substrate. It is also possible for a skin layer otherthan the high crystallinity and low crystallinity layer to be adapted for melt adhesion onto a substrate.

Non-polymeric additives added to either or both layers may include, for example, process oil, flow improvers, fire retardants, antioxidants, plasticizers, pigments, vulcanizing or curative agents, vulcanizing or curative accelerators, cure retarders, processing aids, flame retardants, tackifying resins, and the like. These compounds may include fillers and/or reinforcing materials. These include carbon black, clay, talc, calcium carbonate, mica, silica, silicate, combinations thereof, and the like, Other additives, which may be employed to enhance properties, include antiblocking agents, and a coloring agent. Lubricants, mold release agents, nucleating agents, reinforcements, and fillers (including granular, fibrous, or powder-like) may also be employed. Nucleating agents and fillers tend to improve rigidity of the article. The list described herein is not intended to be inclusive of all types of additives, which may be employed with the present invention.

The overall thickness of the article is not particularly limited, but is typically less than 20 mils or less than 10 mils. The thickness of any of the individual layers is readily determinable by one skilled in the art given the weight percentages discussed herein.

In a particular embodiment, the high crystallinity layer comprises medium or high density polyethylene and the

low crystallinity layer comprises a plastomer. In another particular embodiment, the high crystallinity, layer and low crystallinity layer comprise syndiotactic copolymers having relatively high and low crystallinity. In yet another particular embodiment, the high crystallinity layer comprises isotactic polypropylene and the low crystallinity layer comprises a polypropylene elastomer having relatively low levels of isotactic crystallinity.

Low Crystallinity Layer

The low crystallinity layer has a level of crystallinity that can be detected by Differential Scanning Calorimetry (DSC) but has elastomeric properties. The low crystallinity layer is sufficiently elastic to permit extension of the high crystallinity layer to and beyond the point of plastic deformation without substantial loss of its elastic properties.

The low crystallinity layer comprises a low crystallinity polymer, described in detail below, and optionally at least one additional polymer, described in detail below.

*Low Crystallinity Polymer*

The low crystallinity polymer of the present invention is a soft, elastic polymer with a moderate level of crystallinity due to stereoregular propylene sequences. The low crystallinity polymer can be: (A) a propylene homopolymer in which the stereoregularity is disrupted in some manner such as by regio-inversions; (B) a random propylene copolymer in which the propylene stereoregularity is disrupted at least in part by comonomers; or (C) a combination of (A) and (B).

In a particular embodiment, the low crystallinity polymer is a copolymer of propylene and one or more comonomers selected from ethylene, $C_4$-$C_{12}$ $\alpha$-olefins, and combinations thereof. In a particular aspect of this embodiment, the low crystallinity polymer includes units derived from the one or more comonomers in an amount ranging from a lower limit of 2%, 5%, 6%, 8%, or 10% by weight to an upper limit of 20%, 25%, or 28% by weight. This embodiment will also include propylene-derived units present in an amount ranging from a lower limit of 72%, 75%, or 80% by weight to an upper limit of 98%, 95%, 94%, 92%, or 90% by weight. These percentages by weight are based on the total weight of the propylene-derived and comonomer-derived units; i.e., based on the sum of weight percent propylene-derived units and weight percent comonomer-derived units being 100%.

Embodiments of the invention include low crystallinity polymers having a heat of fusion, as determined by DSC, ranging from a lower limit of 1.0 J/g, or 3.0 J/g, or 5.0 J/g, or 10.0 J/g, or 15.0 J/g, or 20.0 J/g, to an upper limit of 125 J/g, or 100 J/g, or 75 J/g, or 57 J/g, or 50 J/g, or 47 J/g, or 37 J/g, or 30 J/g. As used herein, "heat of fusion" is measured using Differential Scanning Calorimetry (DSC), which can be measured using the ASTM E-794-95 procedure. From 6mg to 10mg of a sheet of the polymer pressed at approximately 200°C to 230°C is removed with a punch die and is annealed at room temperature for 48 hours. At the end of the period, the sample is placed in a Differential Scanning Calorimeter (Perkin Elmer 7 Series Thermal Analysis System) and cooled to -50°C to-70°C. The sample is heated at about 10°C/min to attain a final temperature of 180°C to 200°C. The thermal output is recorded as the area under the melting peak of the sample which is typically at a maximum peak at 30°C to 175°C and occurs between the temperatures of 0°C and 200°C. The thermal output is measured in joules/gram as a measure of the heat of fusion. The melting point is recorded as the temperature of the greatest heat absorption within the range of melting temperature of the sample. Without wishing to be bound by theory, we believe that the low crystallinity polymers of embodiments of our invention have generally isotactic crystallizable propylene sequences, and the above heats of fusion are believed to be due to the melting of these crystalline segments.

The crystallinity of the low crystallinity polymer may also be expressed in terms of crystallinity percent. The thermal energy for the highest order of polypropylene is estimated at 189 J/g. That is, 100% crystallinity is equal to 199 J/g. Therefore, according to the aforementioned heats of fusion, the low crystallinity polymer has a polypropylene crystallinity within the range having an upper limit of 40%, or 30%, or 25%, or 20% and a lower limit of 3%, or 5%, or 7%, or 8%.

The level of crystallinity is also reflected in the melting point. The term "melting point" as used herein is the highest peak among principal and secondary melting peaks as determined by DSC, discussed above. The low crystallinity polymer, according to an embodiment of our invention, has a single melting point. Typically a sample of the polypropylene copolymer will show secondary melting peaks adjacent to the principal peak, which are considered together as a single melting point. The highest of these peaks is considered the melting point. The low crystallinity polymeric has a melting point by DSC ranging from an upper limit of 110°C, or 105°C, or 90°C, or 80°C, or 70°C; to an optional lower limit of 20°C, or 25°C, or 30°C, or 35°C, or 40°C or 45°C.

The low crystallinity polymer can have a weight average molecular weight (Mw) of from 10,000 - 5,000,000 g/mol, or from 20,000 to 1,000,000 g/mol, or from 80,000 to 500,000 g/mol and a molecular weight distribution Mw/Mn (MWD), sometimes referred to as a "polydispersity index" (PDI), ranging from a lower limit of 1.5 or 1.8 to an upper limit of 40 or 20 or 10 or 5 or 3. The Mw and MWD, as used herein, can be determined by a variety of methods, including those in U.S. Patent No. 4,540,753 to Cozewith, et al., and references cited therein, orthose methods found in Verstrate et al., Macromolecules, v. 21, p. 3360 (1988).

In some embodiments of our invention, the low crystallinity, polymer has a Mooney viscosity ML(1+4)@ 125°C of 100 or less, or 75 or less, or 60 or less, or 30 or less. Mooney viscosity, as used herein, can be measured as ML(1+4) @125°C according to ASTM D1646, unless otherwise specified.

The tacticity index, expressed herein as "m/r", is determined by 13C nuclear magnetic resonance (NMR). The tacticity index m/r is calculated as defined in H.N. Cheng, Macromolecules, 17, 1950 (1984). The designation "m" or "r" describes the stereochemistry of pairs of contiguous propylene groups, "m" referring to meso and "r" to racemic. A m/r ratio of 1.0 generally describes a syndiotactic polymer, and an m/r ratio of 2.0 an atactic material. An isotactic material theoretically may have a ratio approaching infinity, and many by-product atactic polymers have sufficient isotactic content to result in ratios of greater than 50. The low crystallinity elastomers used in the invention can have a tacticity index m/r ranging from a lower limit of 4 or 6 to an upper limit of 8 or 10 or 12. An ancillary procedure for the description of the tacticity of the propylene units of embodiments of the current invention is the use of triad tacticity. The triad tacticity of a polymer is the relative tacticity of a sequence of three adjacent propylene units, a chain consisting of head to tail bonds, expressed as a binary combination of m and r sequences. It is usually expressed for copolymers of the present invention as the ratio of the number of units of the specified tacticity to all of the propylene triads in the copolymer.

The triad tacticity (mm fraction) of a propylene copolymer can be determined from a $^{13}C$ NMR spectrum of propylene copolymer and the following formula:

$$\text{mm Fraction} = \frac{\text{PPP(mm)}}{\text{PPP(mm)} + \text{PPP(mr)} + \text{PPP(rr)}}$$

where PPP(mm), PPP(mr) and PPP(rr) denote peak areas derived from the methyl groups of the second units in following three propylene unit chains consisting of head-to-tail bonds:

PPP(mm):

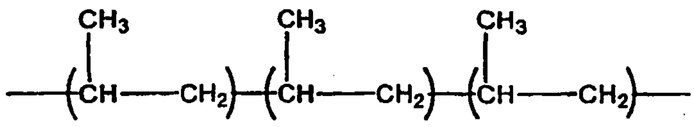

PPP(mr):

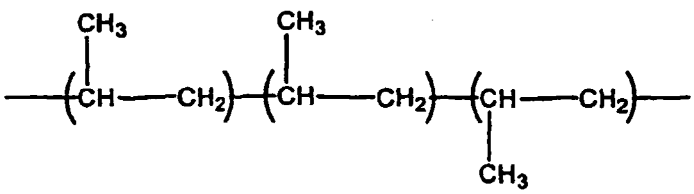

PPP(rr):

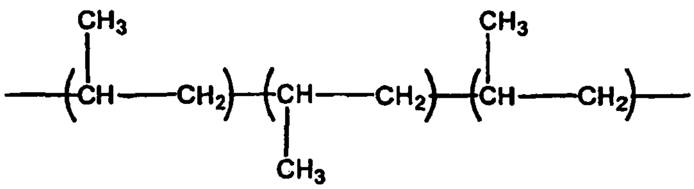

The 13C NMR spectrum of the propylene copolymer is measured as described in U.S. Patent No. 5,504,172. The spectrum relating to the methyl carbon region (19-23 parts per million (ppm)) can be divided into a first region (21.2-21.9 ppm), a second region (20.3-21.0 ppm) and a third region (19.5-20.3 ppm). Each peak in the spectrum was assigned with reference to an article in the journal Polymer, Volume 30 (1989), page 1350. In the first region, the methyl group of the second unit in the three propylene unit chain represented by PPP (mm) resonates. In the second region the methyl group of the second unit in the three propylene unit chain represented by PPP (mr) resonates, and the methyl group (PPE-methyl group) of a propylene unit whose adjacent units are a propylene unit and an ethylene unit resonates (in the vicinity of 20.7 ppm). In the third region, the methyl group of the second unit in the three propylene unit chain

represented by PPP (rr) resonates, and the methyl group (EPE-methyl group) of a propylene unit whose adjacent units are ethylene units resonates (in the vicinity of 19.8 ppm).

The calculation of the triad tacticity is outlined in the techniques shown in U.S. Patent No. 5,504,172. Subtraction of the peak areas for the error in propylene insertions (both 2,1 and 1,3) from peak areas from the total peak areas of the second region and the third region, the peak areas based on the 3 propylene units-chains (PPP(mr) and PPP(rr)) consisting of head-to-tail bonds can be obtained. Thus, the peak areas of PPP(mm), PPP(mr) and PPP(rr) can evaluated, and hence the triad tacticity of the propylene unit chain consisting of head-to-tail bonds can be determined

The low crystallinity polymers of embodiments of our invention have a triad tacticity of three propylene units as measured by 13C NMR, of greater than 75%, or greater than 80%, or greater than 82%, or greater than 85%, greater than 90%.

In one embodiment, the low crystallinity polymer of the present invention includes a random crystallizable copolymer having a narrow compositional distribution. The copolymer is described as random because for a polymer component comprising propylene, minor olefinic comonomer, for example ethylene, and optionally a diene, the number and distribution of ethylene residues is consistent with the random statistical polymerization of the monomers. In stereoblock structures, the number of block monomer residues of any one kind adjacent to one another is greater than predicted from a statistical distribution in random copolymers with a similar composition. Historical ethylene-propylene copolymers with stereoblock structure have a distribution of ethylene residues consistent with these blocky structures rather than a random statistical distribution of the monomer residues in the polymer. The intramolecular composition distribution of the polymer may be determined by 13C NMR. For example, NMR can locate first monomer residues in relation to neighbouring second monomer residues. Furthermore, an evaluation of the randomness of the distribution of sequences may be obtained by the following consideration. We believe that the low crystallinity polymer is random in the distribution of a first and second monomer sequences, such as ethylene and propylene sequences, since (1) it is made with a single sited metallocene catalyst which allows only a single statistical mode of addition of the first and second monomer sequences and (2) it is well mixed in a continuous monomer feed stirred tank polymerization reactor which allows only a single polymerization environment for substantially all of the polymer chains of the low crystallinity, polymer.

The intermolecular composition distribution of a copolymer is determined by thermal fractionation in a solvent. A typical solvent is a saturated hydrocarbon such as hexane or heptane. This thermal fractionation procedure is described below. Typically, approximately 75% by weight and preferably 85% by weight of the polymer is isolated as one or two adjacent, soluble fraction with the balance of the polymer in immediately preceding or succeeding fractions. Each of these fractions has a composition (wt. % ethylene, or other $\alpha$-olefin, content) with a difference of no greater than 20% (relative) and preferably 10% (relative) of the average weight % comonomer, such as ethylene or other $\alpha$-olefin, content of the polypropylene copolymer. The copolymer has a narrow compositional distribution if it meets the fractionation test outlined above.

In one embodiment, the low crystallinity polymer further includes a non-conjugated diene monomer to aid in the vulcanization and other chemical modification of the polymer blend composition. In a particular aspect of this embodiment, the amount of diene can be less than 10 weight %, or less than 5 weight %. The diene may be any non-conjugated diene, which is commonly used for the vulcanization of ethylene propylene rubbers including, but not limited to, ethylidene norbornene, vinyl norbornene, or dicyclopentadiene.

The low crystallinity polymer can be produced by any process that provides the desired polymer properties, in heterogeneous polymerization on a support, such as slurry or gas phase polymerization, or in homogeneous conditions in bulk polymerization in a medium comprising largely monomer or in solution with a solvent as diluent for the monomers. For industrial uses, continuous polymerization processes are preferred. For homogeneous polymers, the polymerization process is preferably a single stage, steady state, polymerization conducted in a well-mixed continuous feed polymerization reactor. The polymerization can be conducted in solution, although other polymerization procedures such as gas phase or slurry polymerization, which fulfill the requirements of single stage polymerization and continuous feed reactors, are contemplated.

The low crystallinity polymers can be made by the continuous solution polymerization process described in WO02/34795 optionally in a single reactor and separated by liquid phase separation from the alkane solvent.

The low crystallinity polymers of the present invention can be produced in the presence of a chiral metallocene catalyst with an activator and optional scavenger. The use of single site catalysts can be used to enhance the homogeneity of the low crystallinity polymer. As only a limited tacticity is needed many different forms of single site catalyst may be used. Possible single site catalysts are metallocenes, such as those described in U.S. Patent No. 5,026,798, which have a single cyclopentadienyl ring, optionally substituted and/or forming part of a polycyclic structure, and a hetero-atom, generally a nitrogen atom, but possibly also a phosphorus atom or phenoxy group connected to a group 4 transition metal, such as titanium, zirconium, or hafnium. A further example is $Me_5CpTiMe_3$ activated with $B(CF)_3$ as used to produce elastomeric polypropylene with an Mn of up to 4 million. See Sassmannshausen, Bochmann, Rosch, Lilge, J. Organomet. Chem. (1997), vol 548, pp. 23-28.

Other possible single site catalysts are metallocenes which are bis cyclopentadienyl derivatives having a group transition metal, such as hafnium or zirconium. Such metallocenes may be unbridged as in U.S. Patent No. 4,522,982 or U.S. Patent No. 5,747,621. The metallocene may be adapted for producing the low crystallinity polymer comprising predominantly propylene derived units as in U.S. Patent No. 5,969,070 which uses an unbridged bis(2-phenyl indenyl) zirconium dichloride to produce a homogeneous polymer having a melting point of above 79°C. The cyclopentadienyl rings may be substituted and/or part of polycyclic systems as described in the above U.S. Patents.

Other possible metallocenes include those in which the two cyclopentadienyls are connected through a bridge, generally a single atom bridge such as a silicon or carbon atom with a choice of groups to occupy the two remaining valencies. Such metallocenes are described in U.S. Patent No. 6,048,950 which discloses bis(indenyl)bis(dimethylsilyl) zirconium dichloride and MAO; WO 98/27154 which discloses a dimethylsilyl bridged bisindenyl hafnium dimethyl together with a non-coordinating anion activator; EP 1070087 which discloses a bridged biscyclopentadienyl catalyst which has elements of asymmetry between the two cyclopentadienyl ligands to give a polymer with elastic properties; and the metallocenes described in U.S. Patent Nos. 6,448,358 and 6,265,212.

The manner of activation of the single site catalyst can vary. Alumoxane, such as methyl alumoxane, may be used. Higher molecular weights may be obtained using non-or weakly coordinating anion activators (NCA) derived and generated in any of the ways amply described in published patent art such as EP277004, EP426637, and many others. Activation generally is believed to involve abstraction of an anionic group such as the methyl group to form a metallocene cation, although according to some literature zwitterions may be produced. The NCA precursor may be an ion pair of a borate or aluminate in which the precursor cation is eliminated upon activation in some manner, e.g. trityl or ammonium derivatives of tetrakis pentafluorophenyl boron (See EP277004). The NCA precursor may be a neutral compound such as a borane, which is formed into a cation by the abstraction of and incorporation of the anionic group abstracted from the metallocene (See EP42663 8).

In a particular embodiment, the low crystallinity polymer is described in detail as the "Second Polymer Component (SPC)" in WO 00/69963, WO 00/01766, WO 99/07788, WO 02/083753, and described in further detail as the "Propylene Olefin Copolymer" in WO 00/01745

Certain specific embodiments described include a copolymer with a specified ethylene composition. The ethylene composition of a polymer can be measured as follows. A thin homogeneous film is pressed at a temperature of 150°C or greater, then mounted on a Perkin Elmer PE 1760 infrared spectrophotometer. A full spectrum of the sample from 600 $cm^{-1}$ to 4000 $cm^{-1}$ is recorded and the monomer weight percent of ethylene can be calculated according to the following equation: Ethylene wt % = 82.585 -111.987X + 30.045 X2, wherein X is the ratio of the peak height at 1155 $cm^{-1}$ and peak height at either 722 $cm^{-1}$ or 732 $cm^{-1}$, whichever is higher. The concentrations of other monomers in the polymer can also be measured using this method.

Comonomer content of discrete molecular weight ranges can be measured by Fourier Transform Infrared Spectroscopy (FTIR) in conjunction with samples collected by GPC. One such method is described in Wheeler and Willis, Applied Spectroscopy, (1993), vol. 47, pp. 1128-1130. Different but similar methods are equally functional for this purpose and well known to those skilled in the art.

Comonomer content and sequence distribution of the polymers can be measured by 13C nuclear magnetic resonance (13C NMR), and such method is well known to those skilled in the art.

In some embodiments, the low crystallinity polymer is present in the article in an amount from a lower limit of 5%, or 10%, or 20%, or 30% or 60% or 70% or 75% to an upper limit of 98%, or 90%, or 85%, or 80%, by weight based on the total weight of the article. The balance of the article includes the high crystallinity polymer, optional additional polymer, and various additives as described above.

*Additional Polymers*

In some embodiments, the low crystallinity layer optionally comprises one or more additional polymers. The optional additional polymer can be the same or different from the high crystallinity polymer of the high crystallinity layer. In a particular embodiment, the additional polymer has a crystallinity between the crystallinity of the low crystallinity polymer and the high crystallinity polymer.

In a particular embodiment, the low crystallinity, layer is a blend comprising a continuous phase including the low crystallinity polymer described above and a dispersed phase including a relatively more crystalline additional polymer. Minor amounts of the additional polymer may be present in the continuous phase. In a particular aspect of this embodiment, the dispersed phase is composed of individual domains less than 50$\mu$m in diameter. In some embodiments, these individual domains of the dispersed phase can be maintained during processing even without cross-linking.

In one embodiment, the additional polymer is a propylene copolymer of ethylene, a $C_4$-$C_{20}$ $\alpha$-olefin, or combinations thereof, wherein the amount of ethylene and/or $C_4$-$C_{20}$ $\alpha$-olefin(s) present in the additional polymer is less than the amount of ethylene and/or $C_4$-$C_{20}$ $\alpha$-olefin(s) present in the low crystallinity polymer. In a particular embodiment, the low crystallinity polymer and additional polymer have polypropylene sequences of the same stereoregularity. In a

non-limiting example, the low crystallinity polymer and the additional polymer include isotactic polypropylene segments, wherein greater than 50% of adjacent polypropylene segments are isotactic.

In one embodiment, the low crystallinity layer is a blend comprising from 2% to about 95% by weight of an additional polymer and from 5% to 98% by weight of the low crystallinity polymer based on the total weight of the blend, wherein the additional polymer is more crystalline than the low crystallinity polymer. In a particular aspect of this embodiment, the additional polymer is present in the blend in an amount of from a lower limit of 2% or 5% to an upper limit of 30% or 20% or 15% by weight based on the total weight of the blend. In another particular aspect of this embodiment, the additional polymer is isotactic polypropylene and has a melting point greater than 110°C, and the low crystallinity polymer is a random copolymer produced by copolymerizing propylene and at least one of ethylene or an alpha-olefin having less than 6 carbon atoms using a chiral metallocene catalyst system. Also, in this embodiment, the low crystallinity polymer has a crystallinity from 2% to 50% from isotactic polypropylene sequences, a propylene content of from 75% to 90% by weight, and a melting point of from 25°C to 105°C.

The blend of the low crystallinity layer is distinguishable from commonly available reactor products, which frequently consist of a blends of isotactic polypropylene and copolymers of propylene and ethylene, which have only a single phase with no prominent dispersed or continuous phases. The present blend is also distinguishable from impact copolymers, thermoplastic olefins, and thermoplastic elastomers produced by chiral metallocene catalysts which when combined with a second polymers have heterophase morphology. Typically, in those materials, the more crystalline polymer is part of the continuous phase and not the dispersed phase. The present blend is also distinguishable from other rnulti-phase blend compositions in that a pre-formed or in situ formed compatibilizer does not need to be added to attain and retain the morphology between the low crystallinity continuous phase and the high crystallinity dispersed phase.

<u>High Crystallinity Layer</u>

The high crystallinity layer has a level of crystallinity sufficient to permit yield and plastic deformation during elongation. The high crystallinity layer can be oriented in a machine direction only or in both a machine and transverse direction as can be detected by microscopy. The orientation can lead to subsequent frangibility of the high crystallinity layer.

The high crystallinity layer includes a high crystallinity polymer. The high crystallinity polymers of the present invention are defined as polymeric components, including blends, that include homopolymer or copolymers of propylene with minor olefinic monomers that include linear, branched, or ring-containing $C_3$ to $C_{30}$ olefins, capable of insertion polymerization, or combinations thereof. In one embodiment, the amount of alpha-olefin in the copolymer has an upper range of 9 wt %, or 8 wt %, or 6 wt%, and a lower range of 2 wt%, based on the total weight of the high crystallinity polymer.

Examples of minor olefinic monomers include, but are not limited to $C_2$ to $C_{20}$ linear or branched $\alpha$-olefins, such as ethylene, propylene, 1-butene, 1-hexene, 1-octene, 4-methyl-1-pentene, 3-methyl-1-pentene, and 3,5,5-trimethyl-1-hexene, and ring-containing olefinic monomers containing up to 30 carbon atoms such as cyclopentene, vinyloyclohexane, vinylcyclohexene, norbornene, and methyl norbornene.

Suitable aromatic-group-containing monomers can contain up to 30 carbon atoms and can comprise at least one aromatic structure, such as a phenyl, indenyl, fluorenyl, or naphthyl moiety. The aromatic-group-containing monomer further includes at least one polymerizable double bond such that after polymerization, the aromatic structure will be pendant from the polymer backbone. The polymerizable olefinic moiety of the aromatic-group containing monomer can be linear, branched, cyclic-containing, or a mixture of these structures. When the polymerizable olefinic moiety contains a cyclic structure, the cyclic structure and the aromatic structure can share 0,1, or 2 carbons. The polymerizable olefinic moiety and/or the aromatic group can also have from one to all of the hydrogen atoms substituted with linear or branched alkyl groups containing from 1 to 4 carbon atoms. Examples of aromatic monomers include, but are not limited to styrene, alpha-methylstyrene, vinyltoluenes, vinyl naphthalene, allyl benzene, and indene, especially styrene and allyl benzene.

In one embodiment, the high crystallinity polymer is a homopolymer orcopolymerof polypropylene with isotactic propylene sequences or mixtures thereof. The polypropylene used can vary widely in form. The propylene component may be a combination of homopolypropylene, and/or random, and/or block copolymers as described herein. In a particular embodiment, the high crystallinity polymer is copolymer of propylene and one or more comonomers selected from ethylene and $C_4$ to $C_{12}$ $\alpha$-olefins. In a particular aspect of this embodiment, the comonomer is present in the copolymer in an amount of up to 9% by weight, or from 2% to 8% by weight, or from 2% to 6% by weight, based on the total weight of the copolymer.

In embodiments of our invention, the high crystallinity polymer has a weight average molecular weight (Mw) of from 10,000 - 5,000,000 g/mol, or from 20,000 to 1,000,000 g/mol, or from 80,000 to 500,000 g/mol and a molecular weight distribution Mw/Mn (sometimes referred to as a "polydispersity index" (PDI)) ranging from a lower limit of 1.5 or 1.8 to an upper limit of 40 or 20 or 10 or 5 or 3.

In one embodiment, the high crystallinity polymer is produced with metallocene catalysis and displays narrow

molecular weight distribution, meaning that the ratio of the weight average molecular weight to the number average molecular weight will be equal to or below 4, most typically in the range of from 1.7 to 4.0, preferably from 1.8 to 2.8.

The high crystallinity polymers of the present invention can optionally contain long chain branches. These can optionally be generated using one or more $\alpha,\omega$ dienes. Alternatively, the high crystallinity polymer may contain small quantities of at least one diene, and preferably at least one of the dienes is a non-conjugated diene to aid in the vulcanization and other chemical modification. The amount of diene is preferably no greater than 10 wt %, more preferably no greater than 5 wt %. Preferred dienes are those that are used for the vulcanization of ethylene propylene rubbers including, but not limited to, ethylidene norbornene, vinyl norbornene, dicyclopentadiene, and 1,4- hexadiene, available from DuPont Chemicals.

Embodiments of our invention include high crystallinity polymers having a heat of fusion, as determined by DSC, with a lower limit of 60 J/g, or 80 J/g. In one embodiment, the high crystallinity polymer has a heat of fusion higher than the heat of fusion of the low crystallinity polymer.

Embodiments of our invention include high crystallinity polymers having a melting point with a lower limit of 100°C, or 110°C, or 115°C, or 120°C, or 130°C.

In one embodiment, the high crystallinity polymer has a higher crystallinity than the low crystallinity polymer. The degree of crystallinity can be determined based on the melting points or the heat of fusion of the polymer components. In one embodiment, the low crystallinity polymer has a lower melting point than the high crystallinity polymer, and the additional polymer, if used, has a melting point between that of the low crystallinity polymer and that of the high crystallinity polymer. In another embodiment, the low crystallinity polymer has a lower heat of fusion than that of the high crystallinity polymer, and the additional polymer, if used, has a heat of fusion intermediate of the low crystallinity polymer and the high crystallinity polymer.

<u>Compatible Crytallinity</u>

The low crystallinity polymer and high crystallinity polymer have compatible stereoregular polypropylene crystallinity. Compatible crystallinity can be obtained by using polymers for the high crystallinity and low crystallinity layers that have the same crystallinity type, i.e., based on the same crystallizable sequence, namely propylene sequences, and the same stereoregular sequences, i.e., isotactic or syndiotactic. Compatible crystallinity is obtained by using polymers with stereoregular propylene sequences. This is achieved, for example, by providing either syndiotactic sequences or isotactic sequences in both layers.

In one embodiment, both the high crystallinity polymer and the low crystallinity polymer, including anything blended in it, contain polypropylene sequences which are substantially isotactic. In another embodiment, both the high crystallinity polymer and the low crystallinity polymer, including anything blended in it, contain polypropylene sequences which are substantially syndiotactic.

Isotactic, as used herein, is defined as referring to a polymer sequence in which greater than 50% of adjacent monomers having groups of atoms that are not part of the backbone structure are located either all above or all below the atoms in the backbone chain, when the latter are all in one plane. Syndiotactic, as used herein, is defined as referring to a polymer sequence in which greater than 50% of adjacent monomers which have groups of atoms that are not part of the backbone structure are located in some symmetrical fashion above and below the atoms in the backbone chain, when the latter are all in one plane.

<u>Applications of the Article</u>

The articles of the present invention may be used in a variety of applications. In one embodiment, the article is a film having at least two layers, which can be used in diaper backsheets and similar absorbent garments such as incontinent garments.

<u>EXAMPLES</u>

Experiments were performed with the following blend components. The low crystallinity polypropylene copolymers containing ethylene as a comonomer are shown in Table 1. These copolymers were produced using a chiral metallocene catalyst known to favor statistically random incorporation of the ethylene comonomer and propylene addition to produce isotactic runs. The copolymer is a thermoplastic elastomer with derived crystallinity resulting from isotactic polypropylene pentads. This copolymer was produced in accordance with the description of the "Second Polymer Component (SPC)" in WO 00/69963 and WO 00/01766.

High crystallinity polymers (HCP) used are polypropylene homopolymers and polyethylene copolymers sold by ExxonMobil Chemical Company, Houston, Texas.

Melt Flow Rate (MFR) and Melt Index (MI), as used herein, were measured by ASTM method D-1238 at 230°C

and 190°C respectively. Mooney Viscosity was measured according to ASTM D1646.

The blends of low crystallinity polymer and high crystallinity polymer and other components may be prepared by any procedure that guarantees an intimate mixture of the components. For example, the components can be combined by melt pressing the components together on a Carver press to a thickness of about 0.5 millimeter (20 mils) and a temperature of about 180°C, rolling up the resulting slab, folding the ends together, and repeating the pressing, rolling, and folding operation about 10 times, Internal mixers are particularly useful for solution or melt blending. Blending at a temperature of about 180°C to 240°C in a Brabender Plastograph for about 1 to 20 minutes has been found satisfactory. Still another method that may be used for admixing the components involves blending the polymers in a Banbury internal mixer above the flux temperature of all of the components, e.g., 180°C for about 5 minutes. A complete mixture of the polymeric components is indicated by the uniformity of the morphology of the dispersion of low crystallinity polymer and high crystallinity polymer. Continuous mixing may also be used. These processes are well known in the art and include single and twin screw mixing extruders, static mixers for mixing molten polymer streams of low viscosity, impingement mixers, as well as other machines and processes, designed to disperse the low crystallinity polymer and the high crystallinity polymer in intimate contact. Those skilled in the art will be able to determine the appropriate procedure for blending of the polymers to balance the need for intimate mixing of the component ingredients with the desire for process economy.

The blend components are selected based on the morphology desired for a given application. The high crystallinity polymer can be co-continuous with the low crystallinity polymer in the film formed from the blend, however, a dispersed high crystallinity polymer phase in a continuous low crystallinity polymer phase is preferred. Those skilled in the art can select the volume fractions of the two components to produce a dispersed high crystallinity polymer morphology in a continuous low crystallinity polymer matrix based on the viscosity ratio of the components (see S. Wu, Polymer Engineering and Science, Vol. 27, Page 335, 1987).

The low crystallinity polymer can be blended with about 10 to 90 weight percent of the high crystallinity polymer, or 15 to 80 weight percent, or 20 to 70 weight percent, based on the total weight of the two polymer components.

Blends were made by mixing all components, including the low crystallinity polymer, the high crystallinity polymer, the optional amounts of process oil and other ingredients in a 2.5" Davis Standard single screw extruder (L/D of 24) under conditions that gave intimate mixing of the components (see Table 3 for typical conditions). The blends were used to make coextruded cast film on a Killion mini cast film line with an ABA structure components (see Table 4 for typical conditions).

The films were allowed to anneal at room temperature for at least 14 days before further processing operations.

Test specimens of the required geometry were removed from the films and evaluated on an Instron 4502 equipped with Test Works Software available from MTS systems, to produce the mechanical deformation data. The Instron Tester and associated equipment is available form The Instron Corporation in Canton, MA. All data is reported in engineering stress and strain terms with values of the stress uncorrected for the contraction in the cross-section of the sample being tested.

As used herein, permanent set can be measured according to the following procedure. The deformable zone (1" wide strip) of the sample was prestretched to 100% of its original length at a deformation rate of 20 in/min. The sample is then relaxed at the same rate. The strain at which no further change in stress is observed is taken to be the permanent set. An alternative way to measure permanent set is to measure the length of the sample that is deformed (D2). The length of the deformation zone in the specimen prior to deformation is measured as D0. The permanent set of the sample is determined by the formula:

$$\text{Permanent set} = 100x(D_2-D_0)/D_0.$$

Load loss was determined on the multi-layer samples, which had been extended on the Instron to 100% extension and then allowed to retract. The stress on loading (at 50% strain) and stress on the unloading cycle (at 50% strain) were noted. The load loss is defined here as:

$$\text{Load loss} = 100*(\text{Stress}_{\text{loading}} - \text{Stress}_{\text{unloading}})/\text{Stress}_{\text{loading}}$$

Table 1 Low crystallinity polymer (LCP) used

| Polymer | ML (1+4) @ 125 °C | MFR | wt%$C_2$ |
|---|---|---|---|
| LCP 1 | 22 | | 17.0 |

(continued)

| Polymer | ML (1+4) @ 125 °C | MFR | wt%$C_2$ |
|---|---|---|---|
| LCP 2 | | 3 | 16.2 |

Table 2 High crystallinity polymers (HCP) used

| Polymer | Sample | MFR | MI |
|---|---|---|---|
| HCP 1 | PP 4292 | 1.5 | |
| HCP 2 | PD 4403 | 7 | |
| HCP 3 | PP 3155 | 36 | |
| HCP 4 | PD 4612E2 | 2.8 | |
| HCP 5* | EXCEED 1018CA | | 1.0 |
| HCP 6 * | EXCEED 1012CA | | 1.0 |
| * only used in comparative examples | | | |

Table 3 Conditions of Davis Standard Extruderto make blends

| Extruder Zones | Temperature (°F) |
|---|---|
| Zone 1 | 320 |
| Zone 2 | 330 |
| Zone 3 | 340 |
| Zone 4 | 350 |
| Zone 5 | 350 |
| Adapter | 370 |
| Screen changer | 380 |
| Adapter | 400 |
| Die | 420 |

Table 4 Conditions on Killion mini cast film line

| | Film 1 | | | Film 2 | | |
|---|---|---|---|---|---|---|
| | A | B | C | A | B | C |
| | LCP1:HCP1 (95:5) | LCP1:HCP1 (95:5) | HCP2 | LCP1:HCP1 (90:10) | LCP1:HCP1 (90:10) | HCP2 |
| Temperature Profile (°F) | | | | | | |
| Zone 1 | 290 | 292 | 385 | 287 | 288 | 386 |
| Zone 2 | 344 | 346 | 404 | 347 | 344 | 404 |
| Zone 3 | 382 | 381 | 419 | 388 | 380 | 419 |
| Adapter 1 | 419 | 420 | 452 | 420 | 419 | 449 |
| Adapter 2 | 420 | 421 | - | 420 | 421 | |
| Die | 450 | - | 450 | 450 | - | 450 |

(continued)

| | Film 1 | | | Film 2 | | |
|---|---|---|---|---|---|---|
| | A | B | C | A | B | C |
| | LCP1:HCP1 (95:5) | LCP1:HCP1 (95:5) | HCP2 | LCP1:HCP1 (90:10) | LCP1:HCP1 (90:10) | HCP2 |
| Melt Temperature (°F) | 394 | 385 | 465 | 399 | 380 | 463 |
| Extruder | | | | | | |
| RPM | 112 | 50 | 15 | 114 | 50 | 15 |
| Pressure (psi) | 2760 | 1010 | 500 | 2770 | 1170 | 510 |
| Drive (amps) | 8 | 2.5 | 2.5 | 7 | 2 | 2.5 |
| Line Speed (fpm) | 6.8 | | | 6.8 | | |
| Chill Roll Temp (°F) | 97 | | | 97 | | |
| Gauge (mil) | 10 | | | 10 | | |
| | Film 1 | | | Film 2 | | |
| | A | B | C | A | B | C |
| | LCP1:HCP1 (95:5) | LCP1:HGP1 (95:5) | HCP2 | LCP1:HCP1 (90:10) | LCP1:HCP1 (90:10) | HCP2 |
| Selector Plug C-B-A-B-C | | | | | | |

The invention, while not meant to be limited thereby, is further illustrated by the following specific examples:

Examples 1-8:

Cast films were made according to Film 1 of Table 4. Specimens of film of 1" width in the form of strips were oriented to different extents along the Machine Direction (MD), in an Instron Tester. The gauge length used was 1" and crosshead speed used was 20"/min. At the end of the orientation, the crosshead returned at the same speed. The specimen was removed, thickness and width remeasured, and then reloaded In the grips of the Instron at a gauge length of 1". The specimen was then elongated to an engineering strain of 100% at a crosshead speed of 20"/min and returned to the original grip spacing of 1" at the same rate. The permanent set and load loss were measured as described earlier.

Table 5 Load loss and permanent set for articles

| Example | Orientation (%) | Load Loss (%) | Permanent Set (%) |
|---|---|---|---|
| 1* | 50 | 85.9 | 17.8 |
| 2* | 100 | 81.7 | 14.0 |
| 3 | 200 | 57.7 | 7.9 |
| 4 | 300 | 49.4 | 7.8 |
| 5 | 500 | 43.8 | 7.3 |
| 6 | 800 | 39.0 | 4.6 |
| 7 | 1000 | 38.6 | 4.5 |
| 8 | 1200 | 39.2 | 4.5 |
| *comparative | | | |

Examples 9-16:

Cast films were made according to Film 2 of Table 4. Specimens of film of 1" width in the form of strips were oriented to different extents along the Machine Direction (MD), in an Instron Tester. The gauge length used was 1" and crosshead speed used was 20"/min. At the end of the orientation, the crosshead returned at the same speed. The specimen was removed, thickness and width remeasured, and then reloaded in the grips of the Instron at a gauge length of 1". The specimen was then elongated to an engineering strain of 100% at a crosshead speed of 20"/min and returned to the original grip spacing of 1" at the same rate. The permanent set and load loss were measured as described earlier.

Table 6 Load loss and permanent set for articles

| Example | Orientation (%) | Load Loss (%) | Permanent Set (%) |
|---|---|---|---|
| 9* | 50 | 81.0 | 14.1 |
| 10* | 100 | 72.5 | 10.5 |
| 11 | 200 | 57.2 | 11.1 |
| 12 | 300 | 49.8 | 7.8 |
| 13 | 500 | 44.6 | 7.8 |
| 14 | 800 | 42.9 | 7.9 |
| 15 | 1000 | 46.0 | 7.3 |
| 16 | 1200 | 46.1 | 7.8 |
| * comparative | | | |

Examples 17-18:

Cast films were made according to Film 1 and Film 2 of Table 4. Samples of dimensions approximately 5 cm x 5 cm were cut from the original films prior to stretching. Specimens were drawn in a T M Long Biaxial stretching machine. Drawing dimensions and conditions are shown in Table 7. After drawing, dogbone specimens of dimensions specified by ASTM D-1708 were punched out of the film samples. These specimens were elongated to an engineering strain of 100% at a crosshead speed of 20"/min and returned to the original grip spacing at the same rate. The permanent set and load loss were measured as described earlier.

Table 7 Stretch conditions, load loss, permanent set and haze for articles

| Examples | 17 | 18 |
|---|---|---|
| Sample | Film 1 | Film 2 |
| Grip pressure (psi) | 400 | 400 |
| Stretching rate (in./sec) | 2 | 1 |
| Temperature (F) | 65 | 65 |
| Stretch in both MD andTD (%) | 400 | 400 |
| Load Loss (%) | 56.8 | 50.3 |
| Permanent Set (%) | 12.4 | 7.7 |
| Haze | 100 | 100 |

Table 8 Roughness measurements for articles

| Example | Sample | $R_a$ ($\mu$m) | $R_y$($\mu$m) | $R_z$($\mu$m) |
|---|---|---|---|---|
| 17 | Film 1 | 1.6 | 9.3 | 4.9 |
| 18 | Film 2 | 2.4 | 14.5 | 7.9 |

Haze and surface roughness of the films were also measured. These are shown in Tables 7 & 8.

Examples 19-26:

Cast films were made according to Films 1 to 8 of Table 9 using a procedure similar to that shown in Table 4. Specimens of film of 1" width in the form of strips were oriented to 400% elongation along the Machine Direction (MD), in an Instron Tester. The gauge length used was 1" and crosshead speed used was 20"/min. At the end of the orientation, the crosshead returned at the same speed. The specimen was removed, thickness and width remeasured, and then reloaded in the grips of the instron at a gauge length of 1". The specimen was then elongated to an engineering strain of 100% at a crosshead speed of 20"/min and returned to the original grip spacing of 1" at the same rate. The permanent set and load loss were measured as described earlier. Haze measurements were made on films before and after stretching, and the data shown in Table 11.

Table 9 Films made on Killion mini cast film line

| | Film 1 | Film 2 | Film 3 | Film 4 | Film 5 | Film 6 | Film 7 | Film 8 |
|---|---|---|---|---|---|---|---|---|
| Extruder A | LCP 2: HCP1 95:5 | LCP 2: HCP1 90:10 | LCP 2: HCP1 85:15 | LCP 2: HCP3 95:5 | LCP 2: HCP3 90:10 | LCP 2: HCP3 85:15 | LCP 2: HCP3 80:20 | LCP 2: HCP3 75:25 |
| Extruder B | LCP2: HCP1 95:5 | LCP2: HCP1 90:10 | LCP2: HCP1 85:15 | LCP 2: HCP3 95:5 | LCP 2: HCP3 90:10 | LCP 2: HCP3 85:15 | LCP 2: HCP3 80:20 | LCP 2: HCP3 75:25 |
| Extruder C | HCP 4 | HCP 4 | HCP 4 | HCP 4 | HCP 4 | HCP 4 | HCP 4 | HCP 4 |
| Gauge (mil) | 6 | 6 | 6 | 6 | 6 | 6 | 6 | 6 |

Table 10 Load loss and permanent set for articles

| Example | Sample | Orientation (%) | Load Loss (%) | Permanent Set (%) |
|---|---|---|---|---|
| 19 | Film 1 | 400 | 57.4 | 14.0 |
| 20 | Film 2 | 400 | 66.2 | 10.7 |
| 21* | Film 3 | 400 | 91.1 | 23.9 |
| 22 | Film 4 | 400 | 54.8 | 11.2 |
| 23 | Film 5 | 400 | 68.4 | 12.3 |
| 24* | Film 6 | 400 | 84.5 | 21.9 |
| 25* | Film 7 | 400 | 91.3 | 22.7 |
| 26* | Film 8 | 400 | 95.6 | 23.8 |
| *comparative | | | | |

Table 11 Haze measurements for articles

| Example | Sample | Haze before Stretching (%) | Haze after Stretching (%) |
|---|---|---|---|
| 19 | Film 1 | 7 | 91 |
| 20 | Film 2 | 16 | 93 |
| 21* | Film 3 | 11 | 90 |
| 22 | Film 4 | 7 | 95 |
| 23 | Film 5 | 9 | 95 |
| 24* | Film 6 | 21 | 93 |

(continued)

| Example | Sample | Haze before Stretching (%) | Haze after Stretching (%) |
|---|---|---|---|
| 25* | Film 7 | 20 | 93 |
| 26* | Film 8 | 14 | 95 |
| *comparative | | | |

Examples 27-33:

Cast films were made according to Films 1 to 8 of Table 9 using a procedure similar to that shown in Table 4. Specimens of film of 1" width in the form of strips were oriented to 400% elongation along the Transverse Direction (TD), in an Instron Tester. The gauge length used was 1" and crosshead speed used was 20"/min, At the end of the orientation, the crosshead returned at the same speed. The specimen was removed, thickness and width remeasured, and then reloaded in the grips of the Instron at a gauge length of 1". The specimen was then elongated to an engineering strain of 100% at a crosshead speed of 20"/min and returned to the original grip spacing of 1" at the same rate. The permanent set and load loss were measured as described earlier. Surface roughness of the films were measured and the data shown in Table 13.

Table 12 Load loss and permanent set for articles

| Example | Sample | Orientation (%) | Load Loss (%) | Permanent Set (%) |
|---|---|---|---|---|
| 27 | Film 1 | 400 | 57.9 | 8.0 |
| 28 | Film 2 | 400 | 58.9 | 9.4 |
| 29* | Film 3 | 400 | 77.3 | 17.3 |
| 30 | Film5 | 400 | 65.3 | 11.2 |
| 31* | Film 6 | 400 | 77.7 | 15.9 |
| 32* | Film 7 | 400 | 86.4 | 17.5 |
| 33* | Film 8 | 400 | 92.6 | 26.0 |
| *comparative | | | | |

Table 13 Roughness measurements for articles

| Example | Sample | $R_a(\mu m)$ | $R_y(\mu m)$ | $R_z(\mu m)$ |
|---|---|---|---|---|
| 27 | Film 1 | 0.6 | 3.5 | 2.1 |
| 28 | Film 2 | 0.8 | 4.4 | 2.9 |
| 29* | Film 3 | 1.3 | 7.7 | 5.3 |
| *comparative | | | | |

Examples 34-41: (Comparative)

Cast films were made according to Films 1 to 8 of Table 14 using a procedure similar to that shown in Table 4. Specimens of film of 1" width in the form of strips were oriented to 400% elongation along the Machine Direction (MD), in an Instron Tester. The gauge length used was 1" and crosshead speed used was 20"/min. At the end of the orientation, the crosshead returned at the same speed. The specimen was removed, thickness and width remeasured, and then reloaded In the grips of the Instron at a gauge length of 1". The specimen was then elongated to an engineering strain of 100% at a crosshead speed of 20"/min and returned to the original grip spacing of 1" at the same rate. The permanent set and load loss were measured as described earlier. Haze and surface roughness measurements were made on some of the films before and after stretching, and the data shown in Tables 16 & 17.

Table 14 Films made on Killion mini cast film line

| | Film 1 | Film 2 | Film 3 | Film 4 | Film 5 | Film 6 | Film 7 | Film 8 |
|---|---|---|---|---|---|---|---|---|
| Extruder A | LCP2: HCP1 95:5 | LCP2: HCP1 90:10 | HCP2: HCP3 95:5 | LCP 2: HCP3 90:10 | LCP2: HCP1 95:5 | LCP2: HCP1 90:10 | LCP 2: HCP3 95:5 | LCP 2: HCP3 90:10 |
| Extruder B | LCP2: HCP1 95:5 | LCP2: HCP1 90:10 | LCP 2: HCP3 95:5 | LCP 2: HCP3 90:10 | LCP2: HCP1 95:5 | LCP 2: HCP1 90:10 | LCP 2: HCP3 95:5 | LCP 2: HCP3 90:10 |
| Extruder C | HCP 5 | HCP 5 | HCP 5 | HCP 5 | HCP 6 | HCP 6 | HCP 6 | HCP 6 |
| Gauge (mil) | 6 | 6 | 6 | 6 | 6 | 6 | 6 | 6 |

Table 15 Load loss and permanent set for articles

| Example | Sample | Orientation (%) | Load Loss (%) | Permanent Set (%) |
|---|---|---|---|---|
| 34 | Film 1 | 400 | 41.9 | 7.5 |
| 35 | Film 2 | 400 | 56.1 | 9.5 |
| 36 | Film 3 | 400 | 39.4 | 4.5 |
| 37 | Film 5 | 400 | 39.9 | 4.2 |
| 38 | Film 6 | 400 | 65.9 | 10.9 |
| 39 | Film 7 | 400 | 37.6 | 4.4 |
| 40 | Film 8 | 400 | 57.9 | 7.6 |

Table 16 Haze measurements for articles

| Example | Sample | Haze before Stretching (%) | Haze after Stretching (%) |
|---|---|---|---|
| 34 | Film 1 | 4 | 98 |
| 35 | Film 2 | 6 | 98 |
| 36 | Film 3 | 2 | 97 |
| 37 | Film 5 | 3 | 96 |
| 38 | Film 6 | 5 | 97 |
| 39 | Film 7 | 2 | 96 |
| 40 | Film 8 | 6 | 97 |

Table 17 Roughness measurements for articles

| Example | Sample | $R_a$ ($\mu$m) | $R_y$ ($\mu$m) | $R_z$ ($\mu$m) |
|---|---|---|---|---|
| 34 | Film 1 | 0.7 | 3.3 | 1.6 |
| 35 | Film 2 | 0.8 | 4.2 | 2.0 |
| 36 | Film 3 | 0.7 | 3.3 | 1.6 |
| 37 | Film 5 | 0.6 | 3.1 | 1.5 |
| 38 | Film 6 | 0.7 | 3.5 | 1.5 |
| 39 | Film 7 | 0.7 | 3.2 | 1.4 |
| 40 | Film 8 | 1.3 | 6.9 | 3.2 |

(continued)

| Example | Sample | $R_a$ (μm) | $R_y$ (μm) | $R_z$ (μm) |
|---|---|---|---|---|
| 41 | Film 8 Before Stretching | 0.4 | 1.6 | 0.7 |

Examples 42-48: (Comparative)

Cast films were made according to Films 1 to 8 of Table 14 using a procedure similar to that shown in Table 4. Specimens of film of 1" width in the form of strips were oriented to 400% elongation along the Transverse Direction (TD), in an Instron Tester. The gauge length used was 1" and crosshead speed used was 20"/min. At the end of the orientation, the crosshead returned at the same speed. The specimen was removed, thickness and width remeasured, and then reloaded in the grips of the instron at a gauge length of 1". The specimen was then elongated to an engineering strain of 100% at a crosshead speed of 20"/min and returned to the original grip spacing of 1" at the same rate. The permanent set and load loss were measured as described earlier. Surface roughness measurements were made on the films and the data shown in Table 18.

Table 18 Load loss and permanent set for articles

| Example | Sample | Orientation (%) | Load Loss (%) | Permanent Set (%) |
|---|---|---|---|---|
| 42 | Film 1 | 400 | 34.7 | 4.2 |
| 43 | Film 2 | 400 | 43.1 | 4.0 |
| 44 | Film 4 | 400 | 50.5 | 4.5 |
| 45 | Film 5 | 400 | 32.9 | 0.9 |
| 46 | Film 6 | 400 | 53.8 | 7.3 |
| 47 | Film 7 | 400 | 37.3 | 4.2 |
| 48 | Film 8 | 400 | 52.7 | 6.1 |

Table 19 Roughness measurements for articles

| Example | Sample | $R_a$ (μm) | $R_y$ (μm) | $R_z$ (μm) |
|---|---|---|---|---|
| 42 | Film 1 | 0.5 | 2.6 | 1.2 |
| 43 | Film 2 | 0.5 | 2.6 | 1.5 |
| 44 | Film 4 | 0.5 | 2.3 | 1.2 |
| 45 | Film 5 | 0.4 | 2.0 | 0.9 |
| 46 | Film 6 | 0.4 | 2.2 | 0.9 |
| 47 | Film 7 | 0.4 | 2.0 | 1.0 |
| 48 | Film 8 | 0.5 | 2.6 | 1.2 |

All patents, test procedures, and other documents cited herein, including priority documents, are fully incorporated by reference to the extent such disclosure is not inconsistent with this invention and for all jurisdictions in which such incorporation is permitted.

While the illustrative embodiments of the invention have been described with particularity, it will be understood that various other modifications will be apparent to and can be readily made by those skilled in the art without departing from the spirit and scope of the invention. Accordingly, it is not intended that the scope of the claims appended hereto be limited to the examples and descriptions set forth herein but rather that the claims be construed as encompassing all the features of patentable novelty which reside in the present invention, including all features which would be treated as equivalents thereof by those skilled in the art to which the invention pertains.

When numerical lower limits and numerical upper limits are listed herein, ranges from any lower limit to any upper limit are contemplated.

## Claims

1. An article comprising:

(a) a low crystallinity layer comprising a low crystallinity polymer, the upper limit of the melting point by DSC of the low crystallinity polymer being 110 °C; and
(b) a high crystallinity layer plastically deformed by elongation comprising a high crystallinity polymer, wherein said high crystallinity polymer has:

a melting point as determined by DSC which is at least 25°C higher than that of said low crystallinity polymer;

wherein said low crystallinity polymer and said high crystallinity polymer have compatible stereoregular polypropylene crystallinity, and said low crystallinity layer is in contact with said plastically deformed high crystallinity layer, and
the article has a Haze value of greater than 70%, and a load loss of less than 70%.

2. The article of claim 1, wherein the low crystallinity layer further comprises an additional polymer which is the same or different from the high crystallinity polymer.

3. The article of claim 2, wherein said additional polymer is different from and more crystalline than said low crystallinity polymer.

4. The article of claim 2, wherein said additional polymer is present in an amount of from 2wt% to 30wt%, preferably 5wt% to 20wt%, based on the total weight of said low crystallinity layer.

5. The article of claim 1, wherein said low crystallinity polymer is a copolymer of propylene and one or more comonomers selected from ethylene and $C_4$-$C_{20}$ $\alpha$-olefins, preferably said one or more comonomers is ethylene, and wherein said one or more comonomers is present in said low crystallinity polymer in an amount of from 2wt% to 25wt%, based on the total weight of said low crystallinity polymer.

6. The article of claim 1, wherein said low crystallinity polymer has a triad tacticity of ≥75%, a narrow compositional distribution, a melting point as determined by DSC of from 25°C to 110°C, or from 35°C to 70°C.

7. The article of claim 1, wherein said low crystallinity polymer has a heat of fusion as determined by DSC of from 3 J/g to 75 J/g.

8. The article of claim 1, wherein said low crystallinity polymer has a molecular weight distribution of from 2.0 to 4.5.

9. The article of claim 1, wherein said high crystallinity polymer is a homopolymer or copolymer of propylene and one or more comonomers selected from ethylene and $C_4$-$C_{12}$ $\alpha$-olefins, preferably said one or more comonomers is ethylene.

10. The article of claim 1, wherein said article comprises an additional layer in contact with said plastically deformed high crystallinity layer.

11. The article of claim 1, wherein said article comprises an additional layer in contact with said low crystallinity layer.

12. The article of claim 11, wherein said additional layer is more crystalline than said low crystallinity layer.

13. The article of claim 11, wherein said additional layer is less crystalline than said low crystallinity layer.

14. The article of claim 1, wherein said article has a Haze value of greater than 80%, or greater than 90%.

15. The article of claim 1, wherein said article has a load loss of less than 60%, or less than 55%.

16. The article of claim 1, wherein said article has a tension set of less than 20%, or less than 15%, or less than 10%.

17. The article of claim 1, wherein said article is a film having two or more layers.

**18.** A garment portion, preferably a diaper backsheet, comprising the article of claim 1 adhered to a garment substrate.

**19.** A process for making the article of any of the preceding claims, said process comprising:

(1) forming said article, and
(2) elongating said article.

**20.** The process of claim 19, wherein said elongating step comprises elongating said article in at least one direction to an elongation of at least 150%, or at least 200%, of its original length or width.

**21.** The process of claim 19, wherein the elongating step comprises elongating the first article in at least one direction to achieve a ∆Haze value of greater than 0%, or at least 10%, or at least 25%, or at least 50%.

## Patentansprüche

**1.** Artikel umfassend:

(a) eine Schicht mit niedriger Kristallinität umfassend ein Polymer mit niedriger Kristallinität, wobei die Obergrenze des Schmelzpunktes mittels DSC des Polymers mit niedriger Kristallinität 110°C beträgt, und
(b) eine Schicht mit hoher Kristallinität, die durch Dehnung plastisch verformt ist, umfassend ein Polymer mit hoher Kristallinität, wobei das Polymer mit hoher Kristallinität aufweist:

einen mittels DSC bestimmten Schmelzpunkt, der mindestens 25°C höher als der des Polymers mit niedriger Kristallinität ist;
wobei das Polymer mit niedriger Kristallinität und das Polymer mit hoher Kristallinität kompatible stereoreguläre Polypropylen-Kristallinität aufweisen und die Schicht mit niedriger Kristallinität in Kontakt mit der plastisch verformten Schicht mit hoher Kristallinität ist und
der Artikel einen Trübungswert von größer als 70% und einen Belastungsverlust von weniger als 70% aufweist.

**2.** Artikel nach Anspruch 1, bei dem die Schicht mit niedriger Kristallinität ferner ein zusätzliches Polymer umfasst, das dasselbe wie das Polymer mit hoher Kristallinität oder davon verschieden ist.

**3.** Artikel nach Anspruch 2, bei dem das zusätzliche Polymer verschieden von und stärker kristallin als das Polymer mit niedriger Kristallinität ist.

**4.** Artikel nach Anspruch 2, bei dem das zusätzliche Polymer in einer Menge von 2 Gew.-% bis 30 Gew.-%, vorzugsweise 5 Gew.-% bis 20 Gew.-%, bezogen auf das Gesamtgewicht der Schicht mit niedriger Kristallinität vorliegt.

**5.** Artikel nach Anspruch 1, bei dem das Polymer mit niedriger Kristallinität ein Copolymer aus Propylen und einem oder mehreren Comonomeren ausgewählt aus Ethylen und $C_4$-$C_{20}$ α-Olefinen ist, wobei vorzugsweise das eine oder mehrere Comonomere Ethylen ist, und wobei das eine oder mehrere Comonomere in dem Polymer mit niedriger Kristallinität in einer Menge von 2 Gew.-% bis 25 Gew.-% bezogen auf das Gesamtgewicht des Polymers mit niedriger Kristallinität vorliegt.

**6.** Artikel nach Anspruch 1, bei dem das Polymer mit niedriger Kristallinität eine Triadentaktizität von ≥ 75%, eine enge Zusammensetzungsverteilung, einen mittels DSC bestimmten Schmelzpunkt von 25°C bis 110°C, oder von 35°C bis 70°C aufweist.

**7.** Artikel nach Anspruch 1, bei dem das Polymer mit niedriger Kristallinität eine mittels DSC bestimmte Schmelzwärme von 3 J/g bis 75 J/g aufweist.

**8.** Artikel nach Anspruch 1, bei dem das Polymer mit niedriger Kristallinität eine Molekulargewichtsverteilung von 2,0 bis 4,5 aufweist.

**9.** Artikel nach Anspruch 1, bei dem das Polymer mit hoher Kristallinität ein Homopolymer oder Copolymer aus Propylen und einem oder mehreren Comonomeren ausgewählt aus Ethylen und $C_4$-$C_{12}$ α-Olefinen ist, wobei vorzugsweise

das eine oder mehrere Comonomere Ethylen ist.

**10.** Artikel nach Anspruch 1, wobei der Artikel eine zusätzliche Schicht in Kontakt mit der plastisch verformten Schicht mit hoher Kristallinität umfasst.

**11.** Artikel nach Anspruch 1, wobei der Artikel eine zusätzliche Schicht in Kontakt mit der Schicht mit niedriger Kristallinität umfasst.

**12.** Artikel nach Anspruch 11, bei dem die zusätzliche Schicht stärker kristallin als die Schicht mit niedriger Kristallinität ist.

**13.** Artikel nach Anspruch 11, bei dem die zusätzliche Schicht weniger kristallin als die Schicht mit niedriger Kristallinität ist.

**14.** Artikel nach Anspruch 1, wobei der Artikel einen Trübungswert von größer als 80%, oder größer als 90% aufweist.

**15.** Artikel nach Anspruch 1, bei dem der Artikel einen Belastungsverlust von weniger als 60%, oder weniger als 55% aufweist.

**16.** Artikel nach Anspruch 1, bei dem der Artikel einen Zugverformungsrest von weniger als 20%, oder weniger als 15%, oder weniger als 10% aufweist.

**17.** Artikel nach Anspruch 1, bei dem der Artikel eine Folie ist, die zwei oder mehr Schichten aufweist.

**18.** Bekleidungsteil, vorzugsweise Windelrückseite, das den Artikel gemäß Anspruch 1 mit einem Bekleidungssubstrat verklebt aufweist.

**19.** Verfahren zur Herstellung des Artikels gemäß einem der vorhergehenden Ansprüche, wobei das Verfahren umfasst:

(1) Bilden des Artikels, und
(2) Dehnen des Artikels.

**20.** Verfahren nach Anspruch 19, bei dem der Dehnungsschritt ein Dehnen des Artikels in mindestens einer Richtung auf eine Dehnung von mindestens 150%, oder mindestens 200%, seiner ursprünglichen Länge oder Breite umfasst.

**21.** Verfahren nach Anspruch 19, bei dem der Dehnungsschritt ein Dehnen des ersten Artikels in mindestens einer Richtung umfasst, um einen ∆Trübungswert von mehr als 0%, oder mindestens 10%, oder mindestens 25%, oder mindestens 50% zu erreichen.

## Revendications

**1.** Article comprenant :

(a)une couche de faible cristallinité comprenant un polymère de faible cristallinité, la limite supérieure du point de fusion par DSC du polymère de faible cristallinité étant 110°C ; et
(b)une couche de cristallinité élevée ayant subi une déformation plastique par allongement comprenant un polymère de cristallinité élevée, ledit polymère de cristallinité élevée possédant :

un point de fusion, déterminé par DSC, qui est supérieur d'au moins 25°C à celui dudit polymère de faible cristallinité ;
ledit polymère de faible cristallinité et ledit polymère de cristallinité élevée ayant la cristallinité compatible d'un polypropylène stéréorégulier, et ladite couche de faible cristallinité étant en contact avec ladite couche de cristallinité élevée ayant subi une déformation plastique, et ledit article possédant une valeur de voile supérieure à 70%, et présentant une perte en charge inférieure à 70%.

**2.** Article selon la revendication 1, dans lequel la couche de faible cristallinité comprend en outre un polymère supplémentaire qui est identique au polymère de cristallinité élevée ou qui est différent.

**3.** Article selon la revendication 2, dans lequel ledit polymère supplémentaire est différent dudit polymère de faible cristallinité et est plus cristallin.

**4.** Article selon la revendication 2, dans lequel ledit polymère supplémentaire est présent à raison de 2% en poids à 30% en poids, de préférence de 5% en poids à 20% en poids, par rapport au poids total de ladite couche de faible cristallinité.

**5.** Article selon la revendication 1, dans lequel ledit polymère de faible cristallinité est un copolymère de propylène et d'un ou plusieurs comonomères choisis parmi l'éthylène et les $\alpha$-oléfines en $C_4$-$C_{20}$, de préférence le ou lesdits comonomères sont de l'éthylène, et dans lequel le ou lesdits comonomères sont présents dans ledit polymère de faible cristallinité à raison de 2% en poids à 25% en poids, par rapport au poids total dudit polymère de faible cristallinité.

**6.** Article selon la revendication 1, dans lequel ledit polymère de faible cristallinité possède une tacticité de triade de $\geq$75%, une distribution de composition étroite, un point de fusion, déterminé par DSC, allant de 25°C à 110°C, ou de 35°C à 70°C.

**7.** Article selon la revendication 1, dans lequel ledit polymère de faible cristallinité possède une chaleur de fusion, déterminée par DSC, allant de 3 J/g à 75 J/g.

**8.** Article selon la revendication 1, dans lequel ledit polymère de faible cristallinité possède une distribution des poids moléculaires allant de 2,0 à 4,5.

**9.** Article selon la revendication 1, dans lequel ledit polymère de cristallinité élevée est un homopolymère ou copolymère de propylène et d'un ou plusieurs comonomères choisis parmi l'éthylène et les $\alpha$-oléfines en $C_4$-$C_{12}$, de préférence le ou lesdits comonomères sont de l'éthylène.

**10.** Article selon la revendication 1, ledit article comprenant une couche supplémentaire en contact avec ladite couche de cristallinité élevée ayant subi une déformation plastique.

**11.** Article selon revendication 1, ledit article comprenant une couche supplémentaire en contact avec ladite couche de faible cristallinité.

**12.** Article selon la revendication 11, dans lequel ladite couche supplémentaire est plus cristalline que ladite couche de faible cristallinité.

**13.** Article selon la revendication 11, dans lequel ladite couche supplémentaire est moins cristalline que ladite couche de faible cristallinité.

**14.** Article selon la revendication 1, ledit article possédant une valeur de voile supérieure à 80%, ou supérieure à 90%.

**15.** Article selon la revendication 1, ledit article présentant une perte en charge inférieure à 60%, ou inférieure à 55%.

**16.** Article la revendication 1, ledit article possédant une déformation rémanente après allongement inférieure à 20%, ou inférieure à 15%, ou inférieure à 10%.

**17.** Article selon la revendication 1, ledit article étant un film possédant deux ou plus de deux couches.

**18.** Pièce de vêtement, de préférence enveloppe extérieure de couche bébé, comprenant l'article selon la revendication 1 collé à un substrat de vêtement.

**19.** Procédé de fabrication de l'article selon l'une quelconque des revendications précédentes, ledit procédé comprenant les étapes consistant à :

(1) former ledit article, et
(2) allonger ledit article.

**20.** Procédé selon la revendication 19, dans lequel ladite étape d'allongement comprend le fait d'allonger ledit article

dans au moins une direction jusqu'à un allongement d'au moins 150%, ou d'au moins 200%, de sa longueur ou de sa largeur initiale.

**21.** Procédé selon la revendication 19, dans lequel l'étape d'allongement comprend le fait d'allonger le premier article dans au moins une direction pour arriver à une valeur de ΔVoile supérieure à 0%, ou d'au moins 10%, ou d'au moins 25%, ou d'au moins 50%.

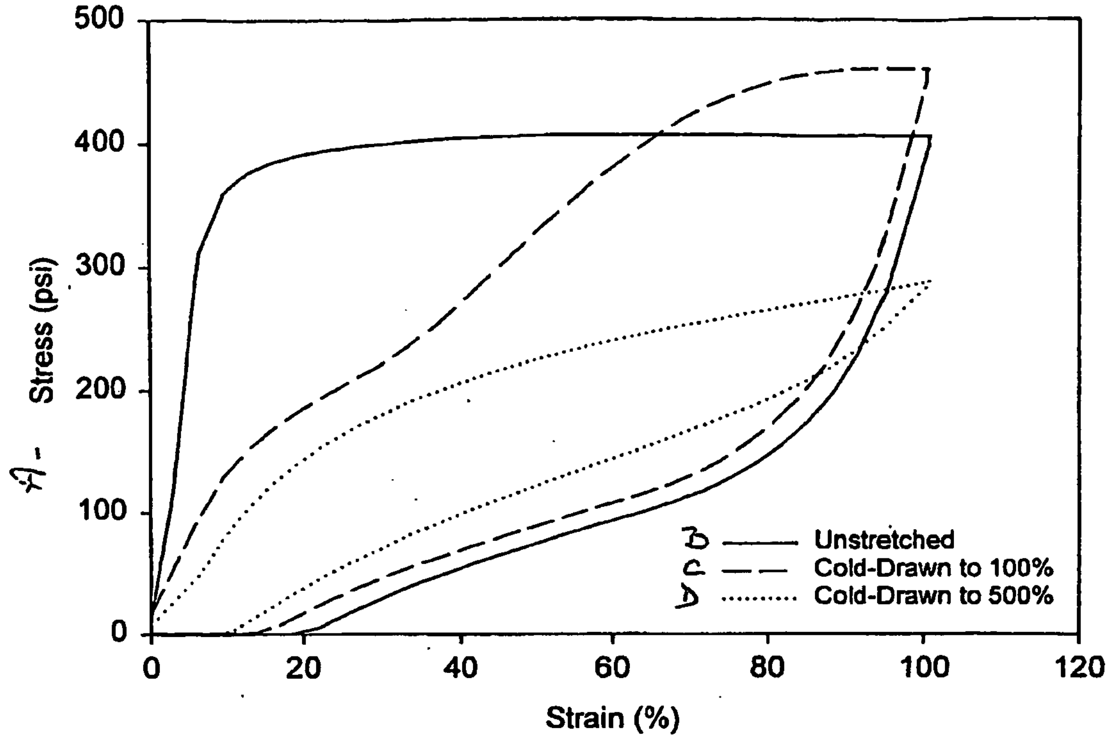
500
400
300
200
100
0
A- Stress (psi)
0
20
40
60
80
100
120
Strain (%)
B Unstretched
C Cold-Drawn to 100%
D Cold-Drawn to 500%

**FIGURE 1**

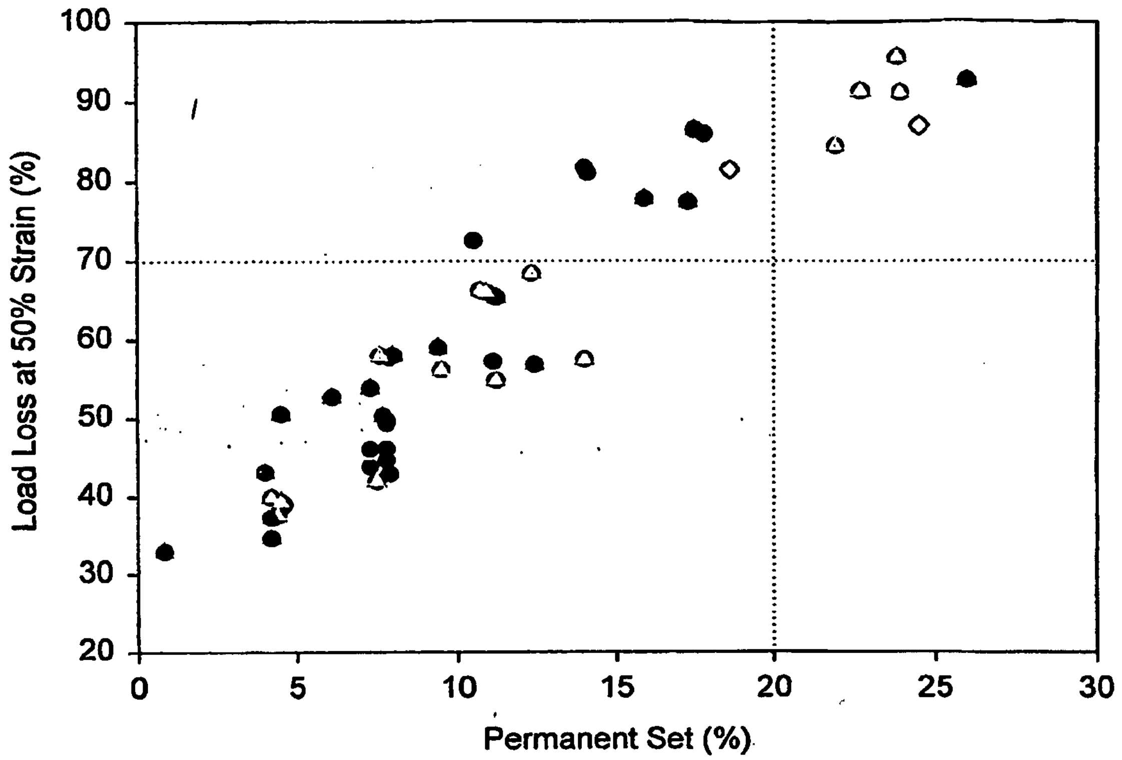

Load Loss at 50% Strain (%)
100
90
80
70
60
50
40
30
20
0
5
10
15
20
25
30
Permanent Set (%)


FIGURE 2

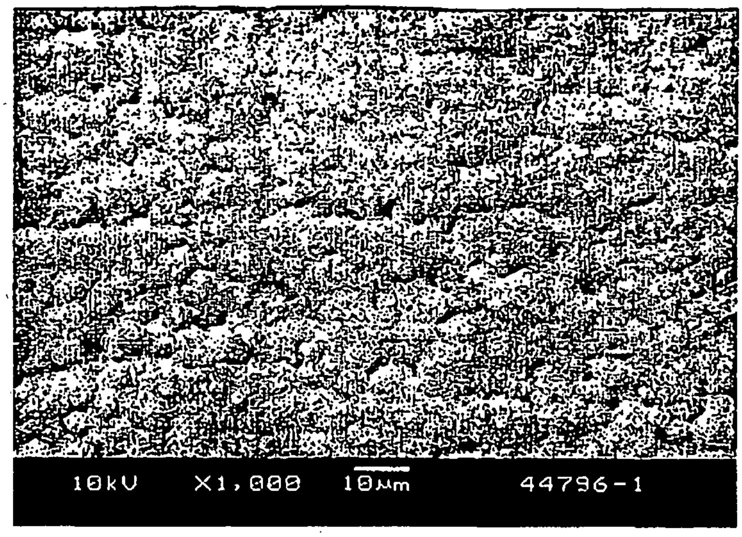


FIGURE 3-A

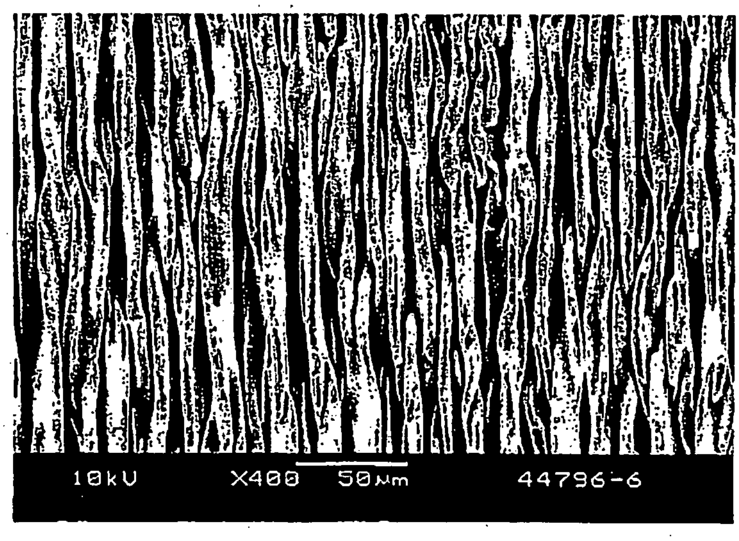


FIGURE 3-B

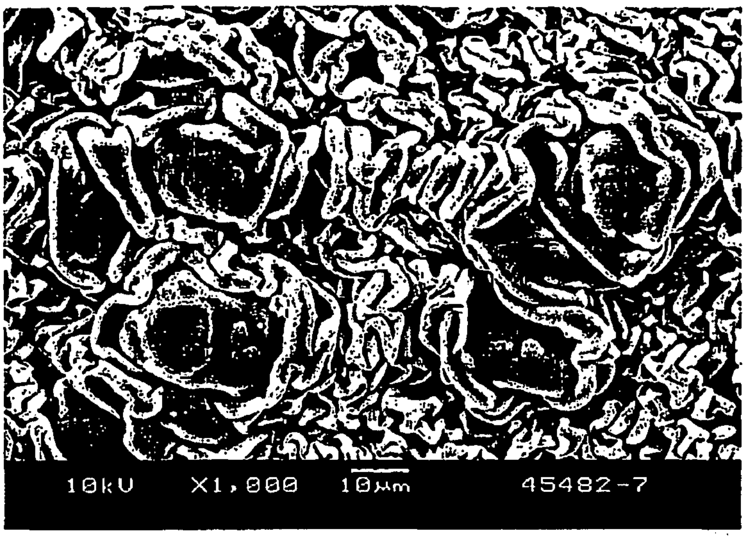


**FIGURE 3-C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6649548 B
- US 4629643 A
- US 5814413 A
- WO 9947339 A
- WO 0105574 A
- US 4714735 A
- US 4820590 A
- US 4880682 A
- US 5354597 A
- US 5376430 A
- US 4476180 A
- US 4540753 A, Cozewith
- US 5504172 A
- WO 0234795 A
- US 5026798 A
- US 4522982 A
- US 5747621 A
- US 5969070 A
- US 6048950 A
- WO 9827154 A
- EP 1070087 A
- US 6448358 B
- US 6265212 B
- EP 277004 A
- EP 426637 A
- EP 426638 A
- WO 0069963 A
- WO 0001766 A
- WO 9907788 A
- WO 02083753 A
- WO 0001745 A


### Non-patent literature cited in the description


- **VERSTRATE et al.** *Macromolecules,* 1988, vol. 21, 3360
- **H.N. CHENG.** *Macromolecules,* 1984, vol. 17, 1950
- *journal Polymer,* 1989, vol. 30, 1350
- **SASSMANNSHAUSEN ; BOCHMANN ; ROSCH ; LILGE.** *J. Organomet. Chem.,* 1997, vol. 548, 23-28
- **WHEELER ; WILLIS.** *Applied Spectroscopy,* 1993, vol. 47, 1128-1130
- **S. WU.** *Polymer Engineering and Science,* 1987, vol. 27, 335